# EUROPÄISCHE PATENTSCHRIFT

(11) **EP 4 190 273 B1**
(45) Veröffentlichungstag und Bekanntmachung des Hinweises auf die Patenterteilung: **27.05.2026**
(21) Anmeldenummer: 22210301.2
(22) Anmeldetag: 29.11.2022
(51) Int. Cl.: A61B 90/57, F16B 2/06, F16B 2/10, F16B 2/18

(54) **SCHNELLSPANNKLEMME**
QUICK-ACTION CLAMPING CLAMP
PINCE À SERRAGE RAPIDE

(30) Priorität: 03.12.2021 DE 102021131909; 22.12.2021 DE 102021134253
(43) Veröffentlichungstag der Anmeldung: 07.06.2023
(73) Patentinhaber: Medizintechnik Sattler GmbH, 07426 Königsee (DE)
(72) Erfinder: Sattler, Sven, 07426 Königsee (DE); Sattler, Danny, 07426 Königsee (DE)
(74) Vertreter: Meissner Bolte Partnerschaft mbB

(56) Entgegenhaltungen:
- EP-A1- 0 972 491
- US-A1- 2005 080 321
- US-A1- 2008 312 509
- US-A1- 2021 330 420

## Beschreibung

Die Erfindung betrifft eine Schnellspannklemme zur Anbringung von chirurgischen Hilfsmitteln wie Wundhaken oder peripherer Operationstechnik an ein Rahmensystem, umfassend zwei mit einer zentralen Aufnahme für einen Zugbolzen versehene Klemmelemente, wobei das erste Klemmelement um die Achse des Zugbolzens drehbar gelagert ist und die Klemmelemente mittels des Zugbolzens, der durch in den Klemmelementen vorhandenen Durchtrittsöffnungen geführt ist, untereinander in mechanischer und drehbarer Wirkverbindung stehen, wobei weiterhin der Zugbolzen mit einem Exzenterhebel über einen Querbolzen in Verbindung steht, der Zugbolzen in einer gabelförmigen Aufnahme des zweiten Klemmelementes verschwenkbar gelagert ist und das an das Rahmensystem koppelbare erste Klemmelement zwei verbundene Schenkel mit einer Klemmöffnung aufweist gemäß Oberbegriff des Anspruches 1.

Aus der US 2009/2106087 A2 ist ein festziehbares Gelenk für ein chirurgisches Rückzugssystem vorbekannt. Das Gelenk umfasst unter anderem eine Klemme zum Aufnehmen einer zu klemmenden Stange. Die Klemme weist eine Schwenkverbindung auf, die einen Kniehebel zeigt.

Aus der EP 3 685 758 A1 sowie der US 10238375 B sind Schnellspannklemmen vorbekannt, die zum Spannen einen Hebel aufweisen, der über einen festen Drehpunkt gelagert ist, wobei die Schwenkbewegung des Hebels über einen Längsschlitz geführt wird.

Schnellspannklemmen, die die Kraftübertragung zum Spannen mittels eines Kniehebels realisieren, sind ebenso aus der EP 2 385 797 B1, der DE 10 2017 100 619 B4 oder der EP 3 287 088 B1 bekannt.

Die Schnellspannklemme nach US 7314331 B1 umfasst zwei Klemmelemente, die mit Hilfe eines Kniehebels unter Nutzung eines Zugbolzens sowie eines Exzenterhebels verspannbar sind.

Bekanntermaßen muss eine Reihe von Operationen im chirurgischen Bereich unter stark vergrößertem Operationsfeld durchgeführt werden. Um derartige Operationsfelder zu erhalten, ist es notwendig, Hautlappen und Gewebe mit Wundhaken zurückzuziehen, um dem Operateur eine freie Sicht auf die geöffneten Körperhöhlen zu gestatten. Wundhaken werden dabei in vielen Fällen von Operationsschwestern oder Assistenzärzten im Verlaufe der Operation gehalten.

Bei langandauernden Operationen stellt dies eine sehr starke körperliche Belastung dar, zumal nicht verhindert werden kann, dass eventuell kleinere Bewegungen des OP-Personals auf den Wundhaken und somit auf das Gewebe übertragen werden.

Diesbezüglich kommen bereits seit längerer Zeit sogenannte Retraktionssysteme zum Einsatz. Retraktionssysteme bestehen aus einem variabel zusammensetzbaren Metallrahmen, an dessen Verbindungsstangen chirurgische Hilfsmittel wie Wundhaken oder periphere Operationstechnik befestigt werden. Zur Anbringung der chirurgischen Hilfsmittel an das Rahmensystem werden Klemmschrauben oder Schnellspannklemmen verwendet.

Bei der EP 2 151 197 B1 wird mit Hilfe einer Schnellspannklemme eine variable und über die vollständige Operationsdauer hinweg sichere Verbindung chirurgischer Hilfselemente mit einem Retraktionssystem bei gleichzeitiger Reduzierung der Anzahl der mechanischen Einzelteile der Klemmvorrichtung geschaffen.

Die vorbekannte Schnellspannklemme umfasst zwei mit einer zentralen Aufnahme für einen Zugbolzen versehene Klemmelemente, wobei das mit dem Rahmensystem zu verbindende Klemmelement zwei miteinander verbundene Schenkel aufweist. Die Klemmelemente stehen dabei mittels eines Zugbolzens, der durch in den Klemmelementen eingearbeitete Durchtrittsöffnungen geführt ist, miteinander in mechanischer Wirkung, wobei an dem zum ersten Klemmelement gegenüberliegenden Ende der Zugbolzen ein Exzenterhebel angeordnet ist.

Das vorbekannte zweite Klemmelement ist als einteiliger Schenkel ausgeführt, der zum Querschnitt eine viertelkreisförmige Aussparung aufweist, wobei der Gegenschenkel durch einen äußeren Oberflächenabschnitt eines der Schenkel des ersten Klemmelementes gebildet ist. Das zweite Klemmelement nimmt ein an das Rahmensystem zu verbindendes chirurgisches Hilfsmittel auf.

In vorteilhafter Weise sind die Durchtrittsöffnungen in der Schenkelbasis des jeweiligen Klemmelementes eingearbeitet und an die jeweilige geometrische Form des entsprechenden Abschnittes des Zugbolzens angepasst, um diesen durch die beiden Klemmelemente führen zu können. Der Einsatz eines Exzenterhebels erlaubt mit geringstem Kraftaufwand eine möglichst große Kraftwirkung auf die Schnellspannklemme, so dass eine Betätigung der Vorrichtung mit nur einer Hand möglich ist.

Um die Hebelkraft des betätigten Exzenterhebels auf das zweite Klemmelement zu übertragen, ist zwischen dem zweiten Klemmelement und dem Exzenterhebel ein Druckstück angebracht, welches in eine Aussparung an der Oberseite des zweiten Klemmelementes gedrückt ist.

Weiterhin ist zwischen dem Druckstück und dem zweiten Klemmelement in einer Aussparung ein federnder O-Ring eingebracht, der vorzugsweise aus medizinischem Silikon gefertigt ist.

Obwohl sich die vorstehend beschriebene Schnellspannklemme im praktischen Einsatz sehr bewährt hat, führt das eingesetzte Druckstück in Verbindung mit dem eingesetzten O-Ring zu Problemen bei dem notwendigen Desinfizieren und Sterilisieren der entsprechenden Klemmen nach deren Einsatz bzw. vor erneuter Verwendung. Vorhandene Spalte sind hier zu klein, um einen ausreichenden Zutritt von Desinfektionsflüssigkeiten zu gewährleisten, jedoch bereits so groß, dass Schmutzpartikel, Reste von Körperflüssigkeiten oder ähnliches eindringen können.

Die US 2008/0312509 A1 offenbart eine Schnellspannklemme zur Anbringung von chirurgischen Hilfsmittel wie Wundhaken oder periphere Operationstechnik an ein Rahmensystem. Unter Anderem zeigt diese Druckschrift einen Zugbolzen, der an einer gabelförmigen Aufnahme eines zweiten Klemmelementes verschwenkbar gelagert ist. Der Zugbolzen ist zweiteilig ausgebildet. Ein erstes senkkopfschraubenartiges Teil ist in einer dem Senkkopf komplementären Ausnehmung im Bodenbereich des ersten Klemmelementes beweglich gelagert. Der senkkopfschraubenartige Teil ist mittels einer Gewindeverbindung von einem hülsenartigen, zweiten Teil aufgenommen, dessen Ende von einem Querbolzen durchdrungen ist. Der Zugbolzen steht mit einem Exzenterhebel über den Querbolzen in Verbindung.

Eine vergleichbare Lösung einer Schnellspannklemme zeigt die US 2021/0330420 A1.

Eine chirurgische Klemme mit einem zweiteiligen Haltebolzen offenbart die US 2005/0080321 A1.

Eine Schnellspannklemme mit einem zweiteiligen Haltebolzen ist auch aus der EP 072491 A1 vorbekannt.

Aus dem Vorgenannten ist es daher Aufgabe der Erfindung, eine weiterentwickelte Schnellspannklemme zur Anbringung von chirurgischen Hilfsmitteln anzugeben, welche eine reduzierte Teileanzahl aufweist, die einfach und intuitiv handhabbar und besonders leicht zu desinfizieren bzw. zu sterilisieren ist.

Die Lösung der Aufgabe der Erfindung erfolgt durch eine Schnellspannklemme gemäß der Merkmalskombination nach Anspruch 1, wobei die Unteransprüche zweckmäßige Ausgestaltungen und Weiterbildungen umfassen.

Ein zusätzliches Druckstück, wie beim eingangs geschilderten Stand der Technik kann hierbei entfallen.

Im entspannten, das heißt im gelösten Zustand der Schnellspannklemme sind alle Oberflächen der Bestandteile der Schnellspannklemme gut zugänglich, so dass eine besonders effektive, leichte und optimale Sterilisation erfolgen kann.

Es wird demnach von einer Schnellspannklemme zur Anbringung von chirurgischen Hilfsmitteln wie Wundhaken oder sonstiger peripherer Operationstechnik an ein Rahmensystem ausgegangen, welche zwei mit einer zentralen Aufnahme für einen Zugbolzen versehene Klemmelemente umfasst.

Das erste Klemmelement ist um die Achse des Zugbolzens drehbar gelagert. Die Klemmelemente sind mittels des Zugbolzens, der durch in den Klemmelementen vorhandene Durchtrittsöffnungen geführt ist, untereinander in mechanischer und drehbarer Wirkverbindung stehend.

Weiterhin steht der Zugbolzen mit einem Spannhebel über einen Querbolzen in Verbindung.

Der Zugbolzen ist in einer gabelförmigen Aufnahme des zweiten Klemmelementes verschwenkbar gelagert.

Das an das Rahmensystem koppelbare erste Klemmelement weist zwei verbundene Schenkel mit einer Klemmöffnung auf.

Weiterhin ist der Zugbolzen zweiteilig ausgebildet.

Ein erstes, senkkopfschraubenartiges Teil des Zugbolzens ist in einer dem Senkkopf komplementären Ausnehmung in einem Bodenbereich des ersten Klemmelementes beweglich gelagert.

Das senkkopfschraubenartige Teil wird mittels einer Gewindeverbindung von einem hülsenartigen, zweiten Teil aufgenommen, dessen Ende vom Querbolzen durchdrungen ist.

Die Durchtrittsöffnungen für den Zugbolzen innerhalb der Klemmteile weisen eine solche lichte Weite und entsprechende Abmessungen auf, die ein ungehindertes Verschwenken des Zugbolzens mittels des Spannhebels von einer Lose-Stellung in eine Kniehebel-Übertotpunkt-Klemmstellung ermöglichen.

Die Gewindeverbindung des zweiteiligen Zugbolzens ist einstellbar, derart, dass in der Lose-Stellung ein Spalt zwischen ersten und zweiten Klemmelement verbleibt.

Die Einstellung der Gewindeverbindung erfolgt üblicherweise herstellerseitig und wird gegen unbefugte Veränderung gesichert.

Die zum ersten Klemmelement gerichtete Oberflächenseite des zweiten Klemmelementes weist in einer Ausgestaltung einen hervorstehenden, umlaufenden Bund auf, welcher in eine komplementäre Führungsöffnung im ersten Klemmelement eintaucht.

Die sich gegenüberliegenden, einander berührenden Oberflächenabschnitte der Klemmenteile können eine Strukturierung, zum Beispiel in Form einer Feinverzahnung, aufweisen, um verschiedene Drehlagepositionen der ersten und zweiten Klemmelemente untereinander im gespannten Zustand sicherzustellen.

Erfindungsgemäß nimmt der Zugbolzen in der Lose-Stellung eine bis zu 7° negative Winkelposition und in der Klemmstellung eine im Wesentlichen 2° positive Winkelposition ein. Die vorstehend erläuterte Winkelposition bezieht sich auf die Zuordnung zu einer im Wesentlichen ebenen Bezugsfläche am ersten Klemmelement. Diese Bezugsfläche befindet sich gegenüber der Klemmöffnung des ersten Klemmelementes.

Die Erfindung soll nachstehend anhand eines Ausführungsbeispieles sowie unter Zuhilfenahme von Figuren näher erläutert werden.

Hierbei zeigen:
- Fig. 1: eine Vorderansicht der Schnellspannklemme im entspannten Zustand;
- Fig. 2: eine Darstellung ähnlich derjenigen nach Fig. 1, jedoch im Zustand einer beginnenden Klemmung;
- Fig. 3: eine Vorderansicht der Schnellspannklemme im Klemmzustand, wobei hier der Spannhebel sich von der Lose-Stellung (Fig. 1) in eine Kniehebel-Übertotpunkt-Klemmstellung befindet bzw. diese Position eingenommen hat;
- Fig. 4: eine Schnittdarstellung längs der Linie N-N;
- Fig. 5: eine Schnittdarstellung gemäß der Linie M-M und

Fig. 6 eine Schnittdarstellung gemäß der Linie L-L (jeweils bezugnehmend auf die angedeuteten Schnitte in den Fig. 1-3).

Die Schnellspannklemme gemäß Ausführungsbeispiel besteht aus einem ersten Klemmelement 1 sowie einem zweiten Klemmelement 2.

Eine zentrale Aufnahme dient der Führung eines Zugbolzens 3.

Das erste Klemmelement 1 ist um die Achse 4 des Zugbolzens 3 drehbar gelagert.

Die Klemmelemente 1; 2 sind mittels des Zugbolzens 3, der durch in den Klemmelementen 1; 2 vorhandenen Durchtrittsöffnungen 5; 6 geführt ist, untereinander in mechanischer und drehbarer Wirkverbindung stehend.

Der Zugbolzen 3 steht mit einem Spannhebel 7 über einen Querbolzen 8 in Verbindung.

Der Zugbolzen 3 ist in einer gabelförmigen Aufnahme 9 des zweiten Klemmelementes 2 verschwenkbar gelagert.

Das erste Klemmelement 1 weist zwei verbundene Schenkel 10; 11 und eine Klemmöffnung 12 sowie eine Bezugsfläche 13 auf.

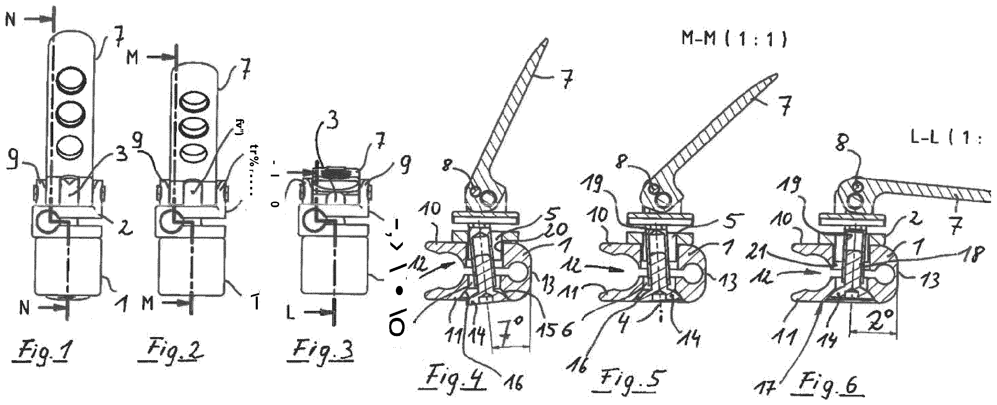

Wie insbesondere aus den Figuren 4 bis 6 erkennbar, ist der Zugbolzen 3 zweiteilig ausgeführt.

Ein erstes, senkkopfschraubenartiges Teil 14 ist in einer dem Senkkopf 15 komplementären Ausnehmung 16 im Bodenbereich 17 des ersten Klemmelementes 1 beweglich, insbesondere verschwenkbar gelagert.

Das senkkopfschraubenartige Teil 14 wird mittels einer Gewindeverbindung 18 von einem hülsenartigen, zweiten Teil 19 des Zugbolzens 3 aufgenommen, dessen Ende vom erwähnten Querbolzen 8 durchdrungen ist.

Erfindungsgemäß sind die Durchtrittsöffnungen 5; 6 für den Zugbolzen 3 innerhalb der Klemmelemente 1; 2 so ausgebildet und weisen eine entsprechende lichte Weite auf, die ein ungehindertes Verschwenken des Zugbolzens 3 mittels des Spannhebels von einer Lose-Stellung in eine Kniehebel-Übertotpunkt-Klemmstellung ermöglicht.

Die Figuren 1 und 4 zeigen die Lose-Stellung.

Die Figuren 3 und 6 die erreichte Kniehebel-Übertotpunkt-Klemmstellung.

Die Gewindeverbindung 18 des zweiteiligen Zugbolzens 3 ist einstellbar, derart, dass in der Lose-Stellung ein Spalt zwischen dem ersten und dem zweiten Klemmelement 1; 2 verbleibt, so dass eine optimale Desinfektion und Sterilisation der Schnellspannklemme erfolgen kann.

Der zum ersten Klemmelement 1 gerichtete Oberflächenabschnitt des zweiten Klemmelementes 2 besitzt einen hervorstehenden Bund 20.

Dieser Bund taucht in eine komplementäre Führungsöffnung 21 im ersten Klemmelement 1 ein.

Die sich gegenüberliegenden, einander im Einsatzfall berührenden Oberflächenabschnitte der Klemmenteile 1; 2 können eine Strukturierung, insbesondere in Form einer Verzahnung oder Feinverzahnung aufweisen (in den Figuren nicht gezeigt).

Die Figur 4 deutet an, dass der Zugbolzen 3 in der Lose-Stellung eine bis zu 7° negative Winkelposition bezogen auf die Bezugsfläche 13 des ersten Klemmelementes einnimmt.

In der Klemmstellung hingegen nimmt der Zugbolzen eine im Wesentlichen 2° positive Winkelposition ein, wiederum im Hinblick auf die Bezugsfläche 13 am ersten Klemmelement 1.

### Bezugszeichenliste

- 1: erstes Klemmelement
- 2: zweites Klemmelement
- 3: Zugbolzen
- 4: Achse des Zugbolzens
- 5; 6: Durchtrittsöffnungen
- 7: Spannhebel
- 8: Querbolzen
- 9: gabelförmige Aufnahme
- 10; 11: Schenkel des ersten Klemmelementes
- 12: Klemmöffnung
- 13: Bezugsfläche am ersten Klemmelement
- 14: senkkopfschraubenartiges erstes Teil
- 15: Senkkopf
- 16: komplementäre Ausnehmung
- 17: Bodenbereich des ersten Klemmelementes
- 18: Gewindeverbindung
- 19: hülsenartiges, zweites Teil
- 20: Bund
- 21: Führungsöffnung

## Patentansprüche

1. Schnellspannklemme zur Anbringung von chirurgischen Hilfsmitteln wie Wundhaken oder peripherer Operationstechnik an ein Rahmensystem, umfassend zwei mit einer zentralen Aufnahme für einen Zugbolzen (3) versehene Klemmelemente (1; 2), wobei das erste Klemmelement (1) um die Achse (4) des Zugbolzens (3) drehbar gelagert ist und die Klemmelemente (1; 2) mittels des Zugbolzens (3), der durch in den Klemmelementen (1; 2) vorhandenen Durchtrittsöffnungen (5; 6) geführt ist, untereinander in mechanischer und drehbarer Wirkverbindung stehen, wobei weiterhin der Zugbolzen (3) mit einem Exzenterhebel (7) über einen Querbolzen (8) in Verbindung steht, der Zugbolzen (3) in einer gabelförmigen Aufnahme (9) des zweiten Klemmelementes (2) verschwenkbar gelagert ist und das an das Rahmensystem koppelbare erste Klemmelement (1) zwei verbundene Schenkel (10; 11) mit einer Klemmöffnung (12) aufweist, wobei
der Zugbolzen (3) zweiteilig ausgebildet ist und ein erstes senkkopfschraubenartiges Teil (14) in einer dem Senkkopf (15) komplementären Ausnehmung (16) in einem Bodenbereich (17) des ersten Klemmelementes (1) beweglich gelagert ist, dass senkkopfschraubenartige Teil (14) mittels einer Gewindeverbindung (18) von einem hülsenartigen, zweiten Teil (19) aufgenommen wird, dessen Ende vom Querbolzen (8) durchdrungen ist und die Durchtrittsöffnungen (5; 6) für den Zugbolzen (3) innerhalb der Klemmteile (1; 2) eine lichte Weite besitzen, welche ein ungehindertes Verschwenken des Zugbolzens (3) mittels des Exzenterhebels (7) von einer Lose-Stellung in eine Kniehebel-Übertotpunkt-Klemmstellung ermöglichen, **dadurch gekennzeichnet, dass**
der Zugbolzen (3) in der Lose-Stellung eine bis zu 7° negative Winkelposition und in der Klemmstellung eine im Wesentlichen 2° positive Winkelposition bezogen auf eine Bezugsfläche (13) parallel zur Durchtrittsöffnung (5) des ersten Klemmelementes (1) einnimmt.

2. Schnellspannklemme nach Anspruch 1,
**dadurch gekennzeichnet, dass**
die Gewindeverbindung (18) des zweiteiligen Zugbolzens (3) einstellbar ist, derart, dass in der Lose-Stellung ein Spalt zwischen ersten und zweiten Klemmelement (1; 2) verbleibt.

3. Schnellspannklemme nach Anspruch 1 oder 2,
**dadurch gekennzeichnet, dass**
die zum ersten Klemmelement (1) gerichtete Oberflächenseite des zweiten Klemmelementes (2) einen hervorstehenden Bund (20) aufweist, welcher in eine komplementäre Führungsöffnung (21) im ersten Klemmelement (1) eintaucht.

4. Schnellspannklemme nach einem der vorangegangenen Ansprüche, **dadurch gekennzeichnet, dass**
die sich gegenüberliegenden, einander berührenden Oberflächenabschnitte der Klemmteile (1; 2) eine Oberflächenstrukturierung aufweisen.

5. Schnellspannklemme nach Anspruch 4,
**dadurch gekennzeichnet, dass**
die Strukturierung als Feinverzahnung ausgebildet ist.

## Claims

1. Quick-action clamp for attaching surgical aids such as retractors or peripheral operation equipment to a frame system, comprising two clamping elements (1; 2) provided with a central receptacle for a tension bolt (3), wherein the first clamping element (1) is mounted to be rotatable around the axis (4) of the tension bolt (3) and the clamping elements (1; 2) have a mechanical and rotatable operational connection to one another by means of the tension bolt (3), which is guided through passage openings (5; 6) present in the clamping elements (1; 2), wherein furthermore the tension bolt (3) is connected to an eccentric lever (7) via a transverse bolt (8), the tension bolt (3) is mounted to be pivotable in a forked receptacle (9) of the second clamping element (2) and the first clamping element (1), which can be coupled to the frame system, has two connected legs (10; 11) having a clamping opening (12),
wherein
the tension bolt (3) is formed in two parts and a first part (14) like a countersunk screw is mounted to be movable in a recess (16) complementary to the countersunk head (15) in a base area (17) of the first clamping element (1),
the part (14) like a countersunk screw is accommodated by means of a threaded connection (18) by a sleeve-like second part (19), the end of which is penetrated by the transverse bolt (8) and the passage openings (5; 6) for the tension bolt (3) within the clamping parts (1; 2) have a clearance which enables unobstructed pivoting of the tension bolt (3) by means of the eccentric lever (7) from a loose position into a toggle lever top dead center clamping position,
**characterized in that** the tension bolt (3) in the loose position adopts an angle position up to -7° and in the clamping position adopts an essentially +2° angle position in relation to a reference surface (13) parallel to the passage opening (5) of the first clamping element (1).

2. Quick-action clamp as claimed in claim 1,
**characterized in that**
the threaded connection (18) of the two-part tension bolt (3) is adjustable such that a gap remains in the loose position between first and second clamping element (1; 2).

3. Quick-action clamp as claimed in claim 1 or 2,
**characterized in that**
the surface side of the second clamping element (2) directed toward the first clamping element (1) has a protruding shoulder (20), which dips into a complementary guide opening (21) in the first clamping element (1).

4. Quick-action clamp as claimed in any one of the preceding claims,
**characterized in that**
the mutually opposing surface sections of the clamping parts (1; 2) which touch one another have a surface structuring.

5. Quick-action clamp as claimed in claim 4,
**characterized in that**
the structuring is formed as fine teeth.

## Revendications

1. Pince à serrage rapide destinée à la fixation d'auxiliaires chirurgicaux, tels que des écarteurs pour plaies ou des moyens techniques opératoires périphériques, sur un système formant cadre, comprenant deux éléments de serrage (1 ; 2) pourvus d'un logement central destiné à recevoir un goujon de traction (3),
dans laquelle
le premier élément de serrage (1) est monté de manière à pouvoir tourner autour de l'axe (4) du goujon de traction (3), et les éléments de serrage (1 ; 2) sont reliés entre eux par une liaison mécanique et rotative au moyen du goujon de traction (3) mené à travers des ouvertures de passage (5 ; 6) présentes dans les éléments de serrage (1 ; 2),
le goujon de traction (3) est en outre relié à un levier excentrique (7) par l'intermédiaire d'un goujon transversal (8), le goujon de traction (3) est monté de manière pivotante dans un logement en forme de fourche (9) du deuxième élément de serrage (2), et le premier élément de serrage (1), pouvant être couplé au système formant cadre, comporte deux branches reliées (10 ; 11) présentant une ouverture de serrage (12),
le goujon de traction (3) est réalisé en deux parties, et une première partie de type vis à tête fraisée (14) est montée de manière mobile dans un évidement (16), complémentaire à la tête fraisée (15), dans une zone de fond (17) du premier élément de serrage (1),
la partie de type vis à tête fraisée (14) est reçue, à l'aide d'un assemblage fileté (18), par une deuxième partie (19) en forme de douille dont l'extrémité est traversée par le goujon transversal (8), et les ouvertures de passage (5 ; 6), destinées à recevoir le goujon de traction (3), à l'intérieur des éléments de serrage (1 ; 2) présentent une largeur intérieure permettant au goujon de traction (3) de pivoter librement, à l'aide du levier excentrique (7), d'une position de desserrage à une position de serrage à genouillère au-delà du point mort,
**caractérisée en ce que**
le goujon de traction (3) adopte, en position de desserrage, une position angulaire négative pouvant aller jusqu'à 7° et, en position de serrage, une position angulaire positive d'environ 2° par rapport à une surface de référence (13) parallèle à l'ouverture de passage (5) du premier élément de serrage (1).

2. Pince à serrage rapide selon la revendication 1,
**caractérisée en ce que**
l'assemblage fileté (18) du goujon de traction en deux parties (3) est réglable de telle sorte qu'un interstice subsiste entre les premier et deuxième éléments de serrage (1 ; 2) dans la position de desserrage.

3. Pince à serrage rapide selon la revendication 1 ou 2,
**caractérisée en ce que**
la face du deuxième élément de serrage (2) tournée vers le premier élément de serrage (1) présente un collet saillant (20) qui s'engage dans une ouverture de guidage complémentaire (21) dans le premier élément de serrage (1).

4. Pince à serrage rapide selon l'une des revendications précédentes,
**caractérisée en ce que**
les parties de surface des éléments de serrage (1 ; 2) qui sont opposées l'une à l'autre et qui se touchent présentent une structuration de surface.

5. Pince à serrage rapide selon la revendication 4,
**caractérisée en ce que**
la structuration est réalisée sous forme de denture fine.
